# EUROPEAN PATENT APPLICATION

(11) **EP 4 023 303 A1**
(43) Date of publication of application: **06.07.2022**
(21) Application number: 20858294.0
(22) Date of filing: 05.08.2020
(51) Int. Cl.: A61Q 5/06, A61K 8/19, A61K 8/22, A61K 8/25, A61K 8/60, A61K 8/9789, A61K 8/9794

(54) **HAIR DYEING METHOD**

(30) Priority: 28.08.2019 JP 2019155296
(71) Applicant: Kotera, Kouichi, Nagoya-shi Aichi, 454-0997 (JP)
(72) Inventor: Kotera, Kouichi, Nagoya-shi Aichi, 454-0997 (JP)
(74) Representative: Lederer & Keller Patentanwälte Partnerschaft mbB
(86) International application number: PCT/JP2020/029938
(87) International publication number: WO 2021/039321

(57) **Abstract**

The present invention provides a hair dyeing method capable of dyeing hair in a variety of colors such as brown and black using a plant dye or natural hair dye that is friendly to the hair and skin without using a diamine-based dye or the like, and of shortening the dyeing time.

The present invention includes an applying step of applying a hair dye liquid to the hair of a subject, a maintaining step of maintaining the hair applied with the hair dye liquid in the state for a predetermined period of time, a rinsing step of rinsing out the hair dye liquid from the hair maintained for the predetermined period of time after application, a color enhancing step of applying a color enhancing agent to the hair with the hair dye liquid rinsed out and then rinsing out the color enhancing agent, and a hair washing step of washing the hair with an oxidative coloring shampoo. The hair dye liquid includes a colorant for plant dyeing such as henna or indigo, a lotion containing polyhydric phenol, a sweating promoter, and sodium bromate. The color enhancing agent contains ferrous ion and bentonite. The oxidative coloring shampoo contains brown sugar, trehalose, potash soap, and sodium bromate.

## Description

### TECHNICAL FIELD

The present invention relates to a hair dyeing method that is employed primarily at beauty salons and the like and, more particularly, to a hair dyeing method using plant dyes that are hair- and skin-friendly.

### BACKGROUND ART

Hair coloring agents used for dyeing graying hair or colored hair may be classified as quasi-drugs and cosmetic products. Generally, permanent hair dyes, in particular oxidative hair dyes (quasi-drugs) are referred to as "hair color", while semi-permanent hair dyes, in particular acidic dyes (cosmetic products) are referred to as "hair manicure". Recently, however, hair dyes (cosmetic products) other than acidic dyes have been referred to as "color rinse" or "color treatment".

These hair coloring agents are distinct in usage or color duration, and a wrong use of hair colors, particularly an oxidative dye and an oxidant in combination, can cause skin troubles such as "dermatitis". Particularly in cases where "dermatitis" by using a hair color is left unattended, the resulting symptom gradually becomes severe and the subsequent sudden, serious allergic reaction of "anaphylaxis" is harmful to the users' health.

A representative ingredient of an oxidative hair dye used in a hair color is para-phenylene diamine, as well as p-aminophenol or toluene-2, 5-diamine (dyes including these substances are hereinafter referred to as "diamine-based dye"). Since these diamine-based dyes act as an allergen, those who have developed "dermatitis" even once cannot use these hair dyes again.

Alternatively, such users dye their hair using plant dyes. Illustrative example of its hair dye includes "henna" and "indigo". In addition, "Ohaguro (or teeth-black-painting)-type hair dyeing" is known not as a typical plant dye but as a similar natural hair dye to produce a colorant in the hair from polyhydric phenol such as ferrous ion and tannin.

The henna is Lythraceae plants that grow naturally from Southwest Asia to North Africa, and its leaves are dried and powdered and such products will be applied to the hair or skin for dyeing. The dyeing effect is produced by a colorant known as "lawsone" contained in henna leaves. In fact, the henna is classified as a permanent hair dye along with hair colors, but its orange-based colorant unfortunately fails to provide stable colors and its dyeing time can be longer.

The indigo is extracted from indigo leaves as polygonaceae plants and is used as a natural dye for dyeing cotton fibers and so forth. Cotton fibers are normally dyed by a dyeing method of chemical treatment using high-alkali dyes with a pH of 13 or more, but this method can seriously damage the hair or skin. Thus, the indigo is used as a hair dye in a method of raw leaf treatment using powders from indigo leaves. The indigo is a blue colorant that fails to provide stable colors, and color development by oxidation coloring needs longer time.

The Ohaguro-type hair dyeing, as described above, allows ferrous ion and polyhydric phenol (tannin or the like) in the hair to produce colorants in the range of red and black to dye hair. This method provides shorter dyeing time and more limited color duration than the hair dyeing using henna or indigo. In addition, the amount of tannin or the like can be controlled to change the color, but the hair tends to be all black or slightly purple, and it is hard to provide stable colors.

Due to these properties, in order to dye hair brown or black, hair dyeing methods are employed by adding indigo having a blue colorant to the orange-based henna or double dyeing of dyeing with henna and subsequently with indigo. The double dyeing unfortunately requires much longer dyeing time of 4 hours or more.

To overcome these drawbacks, henna hair dyes with improved color duration or color development are being proposed in the market. For example, the henna hair dye according to the following Patent Document 1 provides a variety of colors by using a basic dye or HC dye (hair color dye) in combination with the henna.

### CITATION LIST

### PATENT LITERATURE

Patent Document 1: JP-A-2017-186273 A

### SUMMARY OF INVENTION

### TECHNICAL PROBLEM

In fact, a basic dye or HC dye contained in the henna hair dye according to the above Patent Document 1 poses low allergy risk unlike diamine-based dyes acting as an allergen. However, the HC dye is a component that is available after the deregulation of cosmetics ingredients, and the henna hair dye according to the above Patent Document 1 by using the above dyes in combination is totally different from an original plant dye or natural hair dye.

Thus, the present invention was made in view of the situation to solve the problems, and has an object to provide a hair dyeing method capable of dyeing hair in a variety of colors such as brown and black using a plant dye or natural hair dye that is friendly to the hair and skin without using a diamine-based dye or the like, and of shortening the dyeing time.

### SOLUTION TO PROBLEM

To solve the aforementioned problem, inventors of the present invention have carried out an extended investigation to find that use of a plant dye and Ohaguro-type hair dyeing in combination can develop a new applicable color enhancing agent and oxidative coloring shampoo. Based on that technique, the present invention was accomplished.

Specifically, a hair dyeing method according to the present invention is, according to description in claim 1, including:
an applying step of applying a hair dye liquid to the hair of a subject;
a maintaining step of maintaining the hair applied with the hair dye liquid in the state for a predetermined period of time;
a rinsing step of rinsing out the hair dye liquid from the hair maintained for the predetermined period of time after application;
a color enhancing step of applying a color enhancing agent to the hair with the hair dye liquid rinsed out, and then rinsing out the color enhancing agent; and
a hair washing step of washing the hair with an oxidative coloring shampoo, characterized in that
the hair dye liquid is an aqueous solution or a water-soluble cream including a colorant for plant dyeing, a lotion containing polyhydric phenol, a sweating promoter, and sodium bromate,
the color enhancing agent is a paste obtained by kneading a mixture of a powder containing ferrous ion and bentonite and water, and
the oxidative coloring shampoo is a shampoo containing brown sugar, trehalose, potash soap, and sodium bromate.

Furthermore, the present invention is, according to description in claim 2, the hair dyeing method according to claim 1, characterized in that
the color enhancing step is omitted by using the color enhancing agent in combination with the hair dye liquid in the applying step.

Moreover, the present invention is, according to description in claim 3, the hair dyeing method according to claim 1 or 2, characterized in that
the hair dye liquid contains sodium hydrogen carbonate in place of sodium bromate.

Furthermore, the present invention is, according to description in claim 4, the hair dyeing method according to any one of claims 1 to 3, characterized in that
the colorant for plant dyeing contains at least one of henna, indigo, Garcinia, and curcuma.

Moreover, the present invention is, according to description in claim 5, the hair dyeing method according to any one of claims 1 to 4, including
a step of applying a sweating promoter containing a substance for promoting a sweating effect by stimulating capillaries to the scalp and hair of a subject prior to the above applying step of applying the hair dye liquid to the hair.

Furthermore, the present invention is, according to description in claim 6, the hair dyeing method according to any one of claims 1 to 5, characterized in that
the sweating promoter contains at least one of mulberry bark, capsicum, loquat leaf, and Artemisia princeps having an effect of stimulating capillaries.

Moreover, the present invention is, according to description in claim 7, the hair dyeing method according to any one of claims 1 to 6, including
a step of blow-drying, setting and finishing the washed hair after the hair washing step of washing the hair with a shampoo containing the above oxidative coloring agent, characterized in that upon the blow-drying, the colorant is oxidized with air to enhance the color.

### ADVANTAGEOUS EFFECTS OF INVENTION

According to the above configuration, the hair dyeing method of the present invention includes: an applying step of applying a hair dye liquid to the hair of a subject; a maintaining step of maintaining the hair applied with the hair dye liquid in the state for a predetermined period of time; a rinsing step of rinsing out the hair dye liquid from the hair maintained for the predetermined period of time after application; a color enhancing step of applying a color enhancing agent to the hair with the hair dye liquid rinsed out, and then rinsing out the color enhancing agent; and a hair washing step of washing the hair with an oxidative coloring shampoo.

Also, the hair dye liquid is an aqueous solution or a water-soluble cream including a colorant for plant dyeing, a lotion containing polyhydric phenol, a sweating promoter, and sodium bromate. In addition, the color enhancing agent is a paste obtained by kneading a mixture of a powder containing ferrous ion and bentonite and water. Moreover, the oxidative coloring shampoo is a shampoo containing brown sugar, trehalose, potash soap, and sodium bromate.

By using the hair dye liquid, the sweating promoter, and the oxidative coloring shampoo in each of the above steps, a hair dyeing method capable of dyeing hair in a variety of colors such as brown and black using a plant dye or natural hair dye that is friendly to the hair and skin without using a diamine-based dye or the like, and of shortening the dyeing time can be provided.

According to the above configuration, the color enhancing step may be omitted by using the color enhancing agent in combination with the hair dye liquid in the applying step. Accordingly, the above operational advantage can similarly be provided.

According to the above configuration, the hair dye liquid may contain sodium hydrogen carbonate in place of sodium bromate. Accordingly, the above operational advantage can similarly be provided.

According to the above configuration, a colorant for plant dyeing may contain at least one of henna, indigo, Garcinia, and curcuma. Accordingly, the above operational advantage can more specifically be provided.

According to the above configuration, prior to the applying step of applying a hair dye to the hair, a step of applying a sweating promoter containing a substance for promoting a sweating effect by stimulating capillaries to the scalp and hair of a subject may be provided. Accordingly, the above operational advantage can more effectively be provided.

According to the above configuration, a sweating promoter may contain at least one of mulberry bark, capsicum, loquat leaf, and Artemisia princeps having an effect of stimulating capillaries. Accordingly, the above operational advantage can more specifically be provided.

According to the above configuration, after the hair washing step of washing the hair with a shampoo containing an oxidative coloring agent, a step of blow-drying, setting and finishing the washed hair may be provided. Accordingly, upon the blow-drying, the colorant is oxidized with air to enhance the color, and the above operational advantage can more effectively be provided.

### BRIEF DESCRIPTION OF DRAWINGS

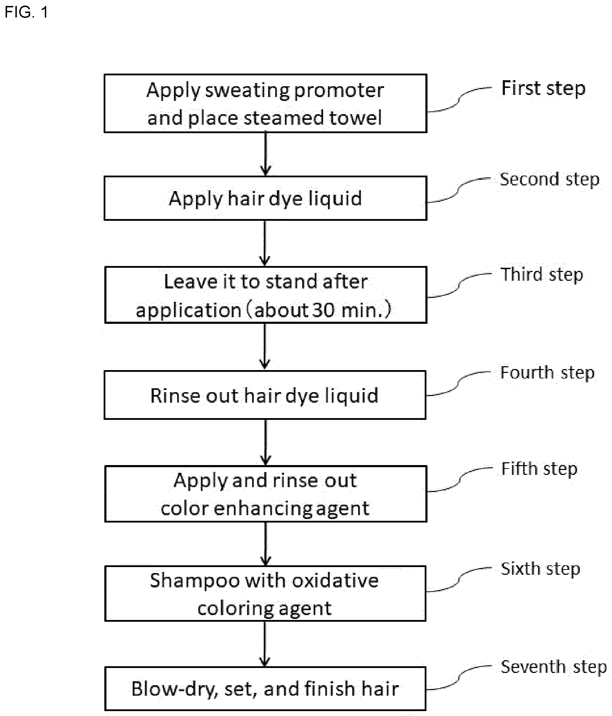

FIG. 1 is a process flow diagram according to one embodiment of the hair dyeing method according to the present invention.

### DESCRIPTION OF EMBODIMENTS

The hair dyeing method according to the present invention will be described with reference to an embodiment. The present invention is not restricted to the following embodiment only.

In the present invention, a new "hair dye liquid", a "lotion containing polyhydric phenol" mixed with the hair dye liquid (hereinafter referred to as "Ohaguro-type tannin lotion"), a "color enhancing agent", an "oxidative coloring shampoo" and the like were developed. A specific "sweating promoter" is also used in combination. A method for preparing these agents will be described. Preparation of each agent used in the present invention is not restricted to the following preparation method only.

The hair dye liquid contains a colorant for plant dyeing, an Ohaguro-type tannin lotion (later-described), a sweating promoter (later-described), and sodium bromate. A color enhancing agent (later-described) may also be used in combination with these agents. Moreover, the above sodium bromate may be replaced with sodium hydrogen carbonate.

The colorant for plant dyeing may be an orange-based colorant "henna", a blue-based colorant "indigo", a yellow-based colorant "Garcinia" or "curcuma", but is not restricted thereto. The Ohaguro-type tannin lotion, as later described, contains polyhydric phenol such as tannin that produces colorants in the range of red and black by reacting with ferrous ion contained in a color enhancing agent. A decoction of "Rhaphiolepis indica" containing tannin used for Oshima Tsumugi weave in textile may also be employed.

The sweating promoter is alkaline to remove hair stain by allowing it to float upward from the hair, and to maintain the pH of the hair optimum for hair dyeing. In addition, the sweating promoter has an effect of maintaining and promoting the health of the hair and skin upon hair dyeing and thereafter. The sodium bromate is an oxidant to mainly promote color development of indigo in this case. The sodium hydrogen carbonate is an alkaline agent to keep alkaline colorants for plant dyeing such as henna and indigo and promote increases in the viscosity of a hair dye liquid itself.

Herein, one example of a method for preparing a hair dye liquid will be described, and the hair dye liquid according to the present invention is not restricted to the following preparation method only. First, colorants for plant dyeing are selected according to the color for hair dyeing. The hair can be dyed black with combined colorants in the range of red and black produced by tannin and ferrous ion using a blue-based colorant, indigo as a hair dye liquid, for example. Meanwhile, the hair can be dyed red or brown with combined colorants in the range of red and black produced by tannin and ferrous ion using an orange-based colorant, henna as a hair dye liquid.

A specific method for preparing a hair dye liquid is to first prepare a mixture of water, an Ohaguro-type tannin lotion, a sweating promoter and 5 % sodium bromate aqueous solution, and mix the same with a henna (or indigo) powder to prepare a creamy hair dye liquid. The hair can be dyed in an intended color by controlling the mixing rate of each of the components. The above 5 % sodium bromate aqueous solution may be replaced with a 5 % sodium hydrogen carbonate aqueous solution.

Subsequently, one example of a method for preparing an Ohaguro-type tannin lotion will be described, and the Ohaguro-type tannin lotion according to the present invention is not restricted to the following preparation method only. A specific preparation method is to mix, for example, 500 ml of 3-year fermented vinegar (red vinegar), 400 ml of pyroligneous acid liquid and 40 g of tartaric acid with 2860 g of water, and feed 240 ml of persimmon juice into the resulting mixture to be mixed well. The mixed liquid is aged for about one month to separate a deposit of a tannin-containing component and water.

The hair can be dyed black, which has conventionally been impossible to achieve, by mixing pyrogallol with the separated tannin-containing component. In addition, the Ohaguro-type tannin lotion according to this embodiment can be used for those with skins too sensitive to persimmon juice used in the conventional Ohaguro-type hair dyeing method.

Subsequently, one example of a method for preparing a color enhancing agent will be described, and the color enhancing agent according to the present invention is not restricted to the following preparation method only. A specific preparation method is to knead a powder obtained by mixing ferrous sulfate and bentonite (volcanic ashes), for example, at a rate of 40:60 with approx. double volume of water, to prepare a color enhancing agent. Herein, it is believed that the purpose of using bentonite is to provide increased viscosity of a paste, a stable oxidative reaction of ferrous sulfate, and an effect of preventing dyeing of the scalp or hands by adsorbing reacted colorants.

Subsequently, one example of a method for preparing an oxidative coloring shampoo will be described, and the oxidative coloring shampoo according to the present invention is not restricted to the following preparation method only. A specific preparation method is to first dissolve 300 g of brown sugar and 60 g of trehalose into 3 L of water to prepare an aqueous solution A. Subsequently, 18 L of potash soap (pure soap content: 35 % fatty acid potassium) and the aqueous solution A are mixed to prepare a mixed liquid B. Subsequently, an aqueous solution C is prepared by dissolving 10 g of sodium bromate into 50 ml of water, and the aqueous solution C and 150 ml of the mixed liquid B are mixed to provide an oxidative coloring shampoo.

The properties of the oxidative coloring shampoo thus prepared lie in better foaming and degradation stability than conventional potash soaps. In the present invention, brown sugar was used in order to improve the foaming property. Trehalose was used to improve the degradation stability.

Subsequently, one example of a method for preparing a sweating promoter will be described, and the sweating promoter according to the present invention is not restricted to the following preparation method only. The sweating promoter according to the present invention contains at least one or all of mulberry bark, capsicum, loquat leaf, and Artemisia princeps having an effect of stimulating capillaries.

Herein, a specific method for preparing a sweating promoter containing all of these ingredients will be described. First, 50 g of mulberry bark, 1 g of capsicum, 15 g of loquat leaf and 20 g of Artemisia princeps are boiled down with 2.2 L of water to obtain 1.95 L of boiled down liquid. With the boiled down liquid were mixed 400 ml of soap (brown sugar shampoo), 500 ml of absolute alcohol, 1000 ml of distilled liquor (25 %), 150 ml of DOL (oil), and 20 g of black salt (rock salt) to obtain a sweating promoter.

Subsequently, one embodiment of the hair dyeing method according to the present invention will be described based on its steps. FIG. 1 is a process flow diagram according to one embodiment of a hair dyeing method according to the present invention.

### <First step>

First, in the first step, a sweating promoter is applied to the scalp and hair of a subject. The method in the present invention doesn't always include a first step of applying a sweating promoter, and such a first step of applying a sweating promoter is preferably performed as a pre-hair dyeing step. A sweating promoter used in the first step is prepared as described above and is alkaline.

As an effect of this first step, an alkaline sweating promoter is preferably used in order to remove hair stain by allowing it to float upward from the hair and then maintain the pH optimum for the hair upon hair dyeing in the following second and subsequent steps. In the first step, the alkaline sweating promoter is uniformly applied to the scalp and hair and a steamed towel is placed entirely on the face and left to stand for a few minutes (preferably for approx. 3 to 5 minutes).

### <Second step>

Subsequently, in the second step, a hair dye liquid is applied to the hair of a subject. A creamy hair dye liquid prepared as described above is applied to parts to be hair-dyed. The application is preferably performed from the root to the tip of the hair.

### <Third step>

Subsequently, in the third step, the hair with the hair dye liquid applied is maintained in the state for a predetermined period of time. A maintaining period of about 30 minutes is sufficient. The maintaining period may be shortened for light-color dyeing. The third step provides light-color dyeing due to insufficient hair dyeing.

### <Fourth step>

Subsequently, in the fourth step, the hair dye liquid is rinsed out from the hair maintained for the predetermined period of time after application. At this time, the hair dye liquid must be sufficiently rinsed out because the residual dye liquid on the scalp or hair of a subject can dye the scalp or forehead, or even hands of a beautician.

### <Fifth step>

Subsequently, in the fifth step, a color enhancing agent is applied uniformly and entirely to the hair with the hair dye liquid rinsed out. Thereafter, the hair is entirely washed by rubbing well and the color enhancing agent is rinsed out after combing. In the fifth step, the color enhancing agent is applied and rinsed out, repeatedly once or twice. In this step, generation of a colorant (iron tannate) can be controlled by reaction of tannin in the hair dye liquid applied to the hair in the second step and ferrous ion in the color enhancing agent applied in the fifth step.

The present invention doesn't always need a fifth step of applying and rinsing out a color enhancing agent. In that case, as described above, a color enhancing agent and sodium hydrogen carbonate are preferably used beforehand in combination with a hair dye liquid in the second step. Accordingly, generation of a colorant (iron tannate) can be controlled by reaction of tannin and ferrous ion not through a fifth step.

### <Sixth step>

Subsequently, in the sixth step, the hair is washed with an oxidative coloring shampoo. In the sixth step, the colorant is subjected to color development and fixation, and the stain that is residual on the scalp or hair of the subject (i.e., color enhancing agent) is sufficiently rinsed out.

### <Seventh step>

Subsequently, in the seventh step, the washed hair is blow-dried, set and finished. At this time, the colorant is oxidized with air even during blow-drying to promote color development. Thus, the hair is preferably not rinsed out after the above sixth step using shampoo, and a small amount of oil is applied to the hair for finishing at the last finishing stage.

Accordingly, since color development is promoted in separate steps, the hair can be dyed by keeping it alkalescent without setting the pH of the hair on the acid side in order to promote oxidation coloring with an oxidant within an applying time, and consequently a hair dyeing agent or colorant can readily be absorbed or adsorbed in the hair. Accordingly, in this embodiment, a hair dyeing step that conventionally requires the time of 4 or more hours can be performed favorably within its half, 2 hours.

As described above, the present invention can provide a hair dyeing method capable of dyeing hair in a variety of colors such as brown and black using a plant dye or natural hair dye that is friendly to the hair and skin without using a diamine-based dye or the like, and of shortening the dyeing time.

The present invention is not restricted to the above embodiment, and they can be replaced with the following various alternatives.

(1) In the hair dyeing method of the above embodiment, the first step is to apply a sweating promoter to the scalp and hair of a subject. However, the hair dyeing method is not restricted thereto, and the method may start with the second step of applying a hair dye liquid to the hair without applying a sweating promoter.
(2) In the hair dyeing method of the above embodiment, a color enhancing agent is applied and rinsed out in the fifth step without using a color enhancing agent in combination with a hair dye liquid in the second step of applying a hair dye liquid to the hair. However, the hair dyeing method is not restricted thereto, and a color enhancing agent may be used in combination with a hair dye liquid in the second step, and a fifth step may be omitted.
(3) In the hair dyeing method of the above embodiment, a hair dye liquid used is sodium bromate, but the liquid is not restricted thereto, and the sodium bromate may be replaced with sodium hydrogen carbonate.

## Claims

1. A hair dyeing method, comprising:
an applying step of applying a hair dye liquid to the hair of a subject;
a maintaining step of maintaining the hair applied with the hair dye liquid in the state for a predetermined period of time;
a rinsing step of rinsing out the hair dye liquid from the hair maintained for the predetermined period of time after application;
a color enhancing step of applying a color enhancing agent to the hair with the hair dye liquid rinsed out, and then rinsing out the color enhancing agent; and
a hair washing step of washing the hair with an oxidative coloring shampoo, wherein
said hair dye liquid is an aqueous solution or a water-soluble cream including a colorant for plant dyeing, a lotion containing polyhydric phenol, a sweating promoter, and sodium bromate,
said color enhancing agent is a paste obtained by kneading a mixture of a powder containing ferrous ion and bentonite and water, and
said oxidative coloring shampoo is a shampoo containing brown sugar, trehalose, potash soap, and sodium bromate.

2. The hair dyeing method according to claim 1, wherein
said color enhancing step is omitted by using said color enhancing agent in combination with said hair dye liquid in said applying step.

3. The hair dyeing method according to claim 1 or 2, wherein
said hair dye liquid contains sodium hydrogen carbonate in place of sodium bromate.

4. The hair dyeing method according to any one of claims 1 to 3, wherein
said colorant for plant dyeing contains at least one of henna, indigo, Garcinia, and curcuma.

5. The hair dyeing method according to any one of claims 1 to 4, comprising a step of applying a sweating promoter containing a substance for promoting a sweating effect by stimulating capillaries to the scalp and hair of a subject prior to the above applying step of applying the hair dye liquid to the hair.

6. The hair dyeing method according to any one of claims 1 to 5, wherein
said sweating promoter contains at least one of mulberry bark, capsicum, loquat leaf, and Artemisia princeps having an effect of stimulating capillaries.

7. The hair dyeing method according to any one of claims 1 to 6, comprising a step of blow-drying, setting and finishing the washed hair after the hair washing step of washing the hair with a shampoo containing the above oxidative coloring agent, wherein
upon the blow-drying, the colorant is oxidized with air to enhance the color.
